(19) **Europäisches Patentamt**
**European Patent Office**
**Office européen des brevets**

(11) **EP 4 382 495 A1**

(12) **EUROPÄISCHE PATENTANMELDUNG**

(43) Veröffentlichungstag:
**12.06.2024 Patentblatt 2024/24**

(21) Anmeldenummer: **22212273.1**

(22) Anmeldetag: **08.12.2022**

(51) Internationale Patentklassifikation (IPC):
**C03C 3/097** (2006.01)     **C03C 4/00** (2006.01)
**C03C 10/04** (2006.01)     **C03C 10/00** (2006.01)

(52) Gemeinsame Patentklassifikation (CPC):
**C03C 3/097; C03C 4/0021; C03C 10/0027**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **Ivoclar Vivadent AG**
**9494 Schaan (LI)**

(72) Erfinder:
- **Rampf, Markus**
  **7212 Seewis-Dorf (CH)**
- **Ritzberger, Christian**
  **9472 Grabs (CH)**
- **Dittmer, Marc**
  **6800 Feldkirch (AT)**

(74) Vertreter: **Uexküll & Stolberg**
**Partnerschaft von**
**Patent- und Rechtsanwälten mbB**
**Beselerstraße 4**
**22607 Hamburg (DE)**

(54) **LITHIUMSILIKAT-GLASKERAMIK MIT LEICHTER BEARBEITBARKEIT**

(57)     Die Erfindung betrifft Lithiumsilikat-Glaskeramik, die Lithiumdisilikat als Hauptkristallphase aufweist und nicht mehr als 40 Gew.-% an Lithiumdisilikat-Kristallen enthält.

EP 4 382 495 A1

Processed by Luminess, 75001 PARIS (FR)

**Beschreibung**

Die Erfindung betrifft Lithiumsilikat-Glaskeramik, die sich vor allem zum Einsatz in der Zahnheilkunde und insbesondere zur Herstellung von dentalen Restaurationen eignet, sowie Vorstufen zur Herstellung dieser Glaskeramik.

Lithiumsilikat-Glaskeramiken zeichnen sich in der Regel durch sehr gute mechanische Eigenschaften aus, weshalb sie seit einiger Zeit im Dentalbereich und dort vornehmlich zur Herstellung von Dentalkronen und kleinen Dentalbrücken Anwendung finden.

WO 95/32678 A2 beschreibt Lithiumdisilikat-Glaskeramiken, die durch Verpressen im viskosen Zustand zu Dentalrestaurationen verarbeitet werden. Dabei ist allerdings zwingend die Verwendung eines deformierbaren Tiegels erforderlich, was die Verarbeitung sehr aufwändig gestaltet.

EP 0 827 941 A1 und EP 0 916 625 A1 offenbaren Lithiumdisilikat-Glaskeramiken, denen durch Verpressen oder maschinelle Bearbeitung die Form der gewünschten Dentalrestauration gegeben werden kann.

EP 1 505 041 A1 und die EP 1 688 398 A1 beschreiben Verfahren zur Herstellung von Dentalrestaurationen aus Lithiumdisilikat-Glaskeramiken. Dabei wird zunächst als Zwischenstufe eine Glaskeramik mit Lithiummetasilikat als Hauptkristallphase erzeugt, die sich mechanisch z.B. mittels CAD/CAM-Verfahren, bearbeiten lässt. Diese Zwischenstufe wird dann einer weiteren Wärmebehandlung unterzogen, um die gewünschte hochfeste Lithiumdisilikat-Glaskeramik zu bilden.

Die maschinelle Bearbeitung von konventionellen Lithiumdisilikat-Glaskeramiken ist aufgrund ihrer hohen Festigkeit nur schwierig möglich und geht daher regelmäßig mit einem hohen Verschleiß der eingesetzten Werkzeuge einher. Eine maschinelle Bearbeitung von Lithiummetasilikat-Glaskeramiken ist grundsätzlich leichter und mit weniger Werkzeugverschleiß möglich. Allerdings müssen die so erhaltenen geformten Lithiummetasilikat-Glaskeramiken einer weiteren Wärmebehandlung unterzogen werden, um Lithiummetasilikat-Kristalle in Lithiumdisilikat-Kristalle umzuwandeln und dadurch Dentalrestaurationen mit ausreichend hoher Festigkeit zu bilden. Dies ist vor allem für die vielfach gewünschte Versorgung eines Patienten mit einer dentalen Restauration in einer einzigen Behandlungssitzung (sogenannte Chairside-Behandlung) problematisch.

Es besteht daher ein Bedarf an Lithiumsilikat-Glaskeramiken, die sich schnell und in einfacher Weise maschinell bearbeiten lassen, die ohne einen weiteren Kristallisationsschritt als dentale Restaurationen eingesetzt werden können und die zudem eine hohe chemische Beständigkeit und ausgezeichnete optische Eigenschaften zeigen.

Diese Aufgabe wird durch die Lithiumsilikat-Glaskeramik nach den Ansprüchen 1 bis 12 und 14 gelöst. Gegenstand der Erfindung sind ebenfalls das Ausgangsglas nach den Ansprüchen 13 und 14, das Verfahren nach den Ansprüchen 15 und 16, die Verwendung nach den Ansprüchen 17 bis 19 sowie das Verfahren nach Anspruch 20.

Die erfindungsgemäße Lithiumsilikat-Glaskeramik zeichnet sich dadurch aus, dass sie Lithiumdisilikat als Hauptkristallphase aufweist und nicht mehr als 40 Gew.-% an Lithiumdisilikat-Kristallen enthält.

Es hat sich überraschenderweise gezeigt, dass die erfindungsgemäße Glaskeramik eine Kombination von sehr wünschenswerten mechanischen und optischen Eigenschaften in sich vereinigt, wie sie gerade für ein restauratives Dentalmaterial erforderlich sind. Diese Glaskeramik hat eine niedrige Festigkeit und Zähigkeit und kann demgemäß leicht und in sehr kurzer Zeit durch maschinelle Bearbeitung in die Form sogar von komplizierten Dentalrestaurationen gebracht werden, kann aber nach einer solchen maschinellen Bearbeitung sogar ohne eine weitere Wärmebehandlung als dentale Restauration mit hervorragenden mechanischen Eigenschaften, ausgezeichneten optischen Eigenschaften und sehr guter chemischer Stabilität eingesetzt werden.

Mit dem Begriff "Hauptkristallphase" wird die Kristallphase bezeichnet, die von allen in der Glaskeramik vorhandenen Kristallphasen den höchsten Massenanteil hat. Die Bestimmung der Massen der Kristallphasen erfolgt dabei insbesondere mit der Rietveld-Methode. Ein geeignetes Verfahren zur quantitativen Analyse der Kristallphasen mittels der Rietveld-Methode ist z.B. in der Dissertation von M. Dittmer "Gläser und Glaskeramiken im System MgO-Al2O3-SiO2 mit ZrO2 als Keimbildner", Universität Jena 2011, beschrieben.

Vorzugsweise enthält die erfindungsgemäße Glaskeramik nicht mehr als 35 Gew.-%, bevorzugt nicht mehr als 32 Gew.-%, weiter bevorzugt nicht mehr als 30 Gew.-% und besonders bevorzugt nicht mehr als 28 Gew.-% an Lithiumdisilikat-Kristallen. Besonders bevorzugt enthält die Glaskeramik 10 bis 40 Gew.-%, bevorzugt 15 bis 35 Gew.-%, weiter bevorzugt 20 bis 32 Gew.-% und besonders bevorzugt 22 bis 30 Gew.-% an Lithiumdisilikat-Kristallen.

Es ist weiter bevorzugt, dass in der erfindungsgemäßen Glaskeramik die Lithiumdisilikatkristalle eine mittlere Länge im Bereich von 10 bis 1000 nm, bevorzugt im Bereich von 50 bis 750 nm, besonders bevorzugt im Bereich von 100 bis 500 nm und ganz besonders bevorzugt im Bereich von 150 bis 250 nm, und ein Aspektverhältnis im Bereich von 1,0 bis 5,0, bevorzugt im Bereich von 1,25 bis 3,0, besonders bevorzugt im Bereich von 1,5 bis 2,5 und ganz besonders bevorzugt im Bereich von 1,75 bis 2,0 aufweisen. Dabei bezeichnet der Begriff "mittlere Länge" das Zahlenmittel der größten Ausdehnung der Kristalle und der Begriff "mittleres Aspektverhältnis" das Zahlenmittel der Quotienten aus größter und kleinster Ausdehnung der Kristalle. Die Messung der Ausdehnungen der Kristalle kann insbesondere anhand von SEM-Aufnahmen, die vorzugsweise an polierten und mit HF-Dampf geätzten Oberflächen der betreffenden Glaskeramik aufgenommen werden, mittels einer Bildanalysesoftware wie etwa der Software Olympus Stream Motion

EP 4 382 495 A1

(Olympus Corporation, Tokyo, Japan) erfolgen.

Die erfindungsgemäße Lithiumsilikat-Glaskeramik enthält insbesondere 62,0 bis 80,0, bevorzugt 64,0 bis 75,0 und besonders bevorzugt 65,0 bis 73,0 Gew.-% $SiO_2$.

Es ist weiter bevorzugt, dass die Glaskeramik 7,0 bis 13,0, bevorzugt 9,0 bis 12,5 und besonders bevorzugt 10,0 bis 12,0 Gew.-% $Li_2O$ enthält. Es wird angenommen, dass $Li_2O$ die Viskosität der Glasmatrix erniedrigt und damit die Kristallisation der gewünschten Phasen fördert.

In einer weiteren bevorzugten Ausführungsform enthält die Glaskeramik 2,0 bis 12,0, bevorzugt 3,0 bis 10,0 und besonders bevorzugt 5,0 bis 9,0 Gew.-% weiteres Oxid einwertiger Elemente $Me^I_2O$, wobei $Me^I_2O$ aus $Na_2O$, $K_2O$, $Rb_2O$, $Cs_2O$ und Mischungen davon ausgewählt ist und vorzugsweise $K_2O$ ist.

Besonders bevorzugt enthält die Glaskeramik mindestens eines und insbesondere alle der folgenden weiteren Oxide einwertiger Elemente $Me^I_2O$ in den angegebenen Mengen:

| Komponente | Gew.-% |
|---|---|
| $Na_2O$ | 0 bis 5, 0 |
| $K_2O$ | 0 bis 10,0 |
| $Rb_2O$ | 0 bis 8,0 |
| $Cs_2O$ | 0 bis 8,0. |

In einer besonders bevorzugten Ausführungsform enthält die erfindungsgemäße Glaskeramik 2,0 bis 10,0, bevorzugt 3,0 bis 9,0 und besonders bevorzugt 5,0 bis 8,0 Gew.-% $K_2O$.

Auch ist es bevorzugt, dass die Glaskeramik 3,0 bis 12,0, bevorzugt 3,0 bis 10,0, besonders bevorzugt 3,0 bis 9,0 und ganz besonders bevorzugt 4,0 bis 7,0 Gew.-% $Al_2O_3$ enthält.

In einer weiteren bevorzugten Ausführungsform enthält die Glaskeramik 0,5 bis 10,0, bevorzugt 1,0 bis 8,0, weiter bevorzugt 1,5 bis 6,0, besonders bevorzugt 1,8 bis 5,0 und ganz besonders bevorzugt 2,0 bis 3,0 Gew.-% $P_2O_5$. Es wird angenommen, dass das $P_2O_5$ als Keimbildner wirkt.

Des Weiteren ist es bevorzugt, dass die Glaskeramik 0 bis 8,0, bevorzugt 0,5 bis 7,0, besonders bevorzugt 1,0 bis 6,0 und ganz besonders bevorzugt 2,0 bis 4,0 Gew.-% Oxid zweiwertiger Elemente $Me^{II}O$ ausgewählt aus der Gruppe von MgO, CaO, SrO, ZnO und Mischungen davon enthält.

In einer weiteren bevorzugten Ausführungsform enthält die Glaskeramik weniger als 4,0, bevorzugt weniger als 2,0 und besonders bevorzugt weniger als 1,0 Gew.-% an BaO. Die Glaskeramik ist insbesondere im Wesentlichen frei von BaO.

Bevorzugt enthält die Glaskeramik mindestens eines und insbesondere alle der folgenden Oxide zweiwertiger Elemente $Me^{II}O$ in den angegebenen Mengen:

| Komponente | Gew.-% |
|---|---|
| MgO | 0 bis 4,0 |
| CaO | 0 bis 4,0 |
| SrO | 0 bis 10,0 |
| ZnO | 0 bis 6,0. |

In einer besonders bevorzugten Ausführungsform enthält die Glaskeramik 0 bis 4,0, bevorzugt 0,5 bis 3,5 und besonders bevorzugt 1,0 bis 3,0 Gew.-% MgO.

In einer weiteren besonders bevorzugten Ausführungsform enthält die Glaskeramik 0 bis 10,0, bevorzugt 0,5 bis 8,0, besonders bevorzugt 1,0 bis 6,0 und ganz besonders bevorzugt 2,0 bis 4,0 Gew.-% SrO.

Es ist weiter eine Glaskeramik bevorzugt, die 0 bis 12,0, bevorzugt 0,5 bis 10,0, besonders bevorzugt 1,0 bis 8,0 und besonders bevorzugt 2,0 bis 6,0 Gew.-% weiteres Oxid dreiwertiger Elemente $Me^{III}_2O_3$ ausgewählt aus der Gruppe von $B_2O_3$, $Y_2O_3$, $La_2O_3$, $Ga_2O_3$, $In_2O_3$ und Mischungen davon enthält.

Besonders bevorzugt enthält die Glaskeramik mindestens eines und insbesondere alle der folgenden weiteren Oxide dreiwertiger Elemente $Me^{III}_2O_3$ in den angegebenen Mengen:

| Komponente | Gew.-% |
|---|---|
| $B_2O_3$ | 0 bis 8,0 |
| $Y_2O_3$ | 0 bis 8,0 |

3

(fortgesetzt)

| Komponente | Gew.-% |
|---|---|
| $La_2O_3$ | 0 bis 8,0 |
| $Ga_2O_3$ | 0 bis 5, 0 |
| $In_2O_3$ | 0 bis 5,0. |

Ferner ist eine Glaskeramik bevorzugt, die 0 bis 12,0, bevorzugt 0,5 bis 10,0 und besonders bevorzugt 1,0 bis 9,0 Gew.-% Oxid vierwertiger Elemente $Me^{IV}O_2$ ausgewählt aus der Gruppe von $TiO_2$, $ZrO_2$, $GeO_2$, $SnO_2$, $CeO_2$ und Mischungen davon enthält.

Besonders bevorzugt enthält die Glaskeramik mindestens eines und insbesondere alle der folgenden Oxide vierwertiger Elemente $Me^{IV}O_2$ in den angegebenen Mengen:

| Komponente | Gew.-% |
|---|---|
| $TiO_2$ | 0 bis 5,0 |
| $ZrO_2$ | 0 bis 12,0 |
| $GeO_2$ | 0 bis 5,0 |
| $SnO_2$ | 0 bis 5, 0 |
| $CeO_2$ | 0 bis 5,0 |

In einer besonders bevorzugten Ausführungsform enthält die Glaskeramik 0 bis 12,0 bevorzugt 1,0 bis 10,0 und besonders bevorzugt 4,0 bis 8,0 Gew.-% $ZrO_2$.

In einer weiteren Ausführungsform enthält die Glaskeramik 0 bis 10,0, bevorzugt 1,0 bis 9,0 und besonders bevorzugt 2,0 bis 8,0 Gew.-% weiteres Oxid fünfwertiger Elemente $Me^{V}_2O_5$ ausgewählt aus der Gruppe von $V_2O_5$, $Nb_2O_5$, $Ta_2O_5$ und Mischungen davon.

Besonders bevorzugt enthält die Glaskeramik mindestens eines und insbesondere alle der folgenden weiteren Oxide fünfwertiger Elemente $Me^{V}_2O_5$ in den angegebenen Mengen:

| Komponente | Gew.-% |
|---|---|
| $V_2O_5$ | 0 bis 2, 0 |
| $Nb_2O_5$ | 0 bis 10,0 |
| $Ta_2O_5$ | 0 bis 10,0 |

In einer weiteren Ausführungsform enthält die Glaskeramik 0 bis 8,0, bevorzugt 1,0 bis 6,0 und besonders bevorzugt 2,0 bis 4,0 Gew.-% Oxid sechswertiger Element $Me^{VI}O_3$ ausgewählt aus der Gruppe von $MoO_3$, $WO_3$ und Mischungen davon.

Besonders bevorzugt enthält die Glaskeramik mindestens eines und insbesondere alle der folgenden Oxide $Me^{VI}O_3$ in den angegebenen Mengen:

| Komponente | Gew.-% |
|---|---|
| $MoO_3$ | 0 bis 3,0 |
| $WO_3$ | 0 bis 3,0. |

In einer weiteren Ausführungsform enthält die erfindungsgemäße Glaskeramik 0 bis 5,0, bevorzugt 0,1 bis 2,0 und besonders bevorzugt 0,5 bis 1,0 Gew.-% Fluor.

Besonders bevorzugt ist eine Glaskeramik, die mindestens eine und bevorzugt alle der folgenden Komponenten in den angegebenen Mengen enthält:

| Komponente | Gew.-% |
|---|---|
| $SiO_2$ | 62,0 bis 80,0 |
| $Li_2O$ | 7,0 bis 13,0 |

(fortgesetzt)

| Komponente | Gew.-% |
|---|---|
| $Me^I_2O$ | 2,0 bis 12,0, insbesondere 5,0 bis 10,0 |
| $Al_2O_3$ | 3,0 bis 12,0 |
| $P_2O_5$ | 0,5 bis 10,0 |
| $Me^{II}O$ | 0 bis 8,0 |
| $Me^{III}_2O_3$ | 0 bis 12,0 |
| $Me^{IV}O_2$ | 0 bis 12, 0 |
| $Me^V_2O_5$ | 0 bis 10, 0 |
| $Me^{VI}O_3$ | 0 bis 8,0 |
| Fluor | 0 bis 5,0, |

wobei $Me^I_2O$, $Me^{II}O$, $Me^{III}_2O_3$, $Me^{IV}O_2$, $Me^V_2O_5$ und $Me^{VI}O_3$ die oben angegebenen Bedeutungen haben.

In einer weiteren besonders bevorzugten Ausführungsform enthält die Glaskeramik mindestens eine und bevorzugt alle der folgenden Komponenten in den angegebenen Mengen:

| Komponente | Gew.-% |
|---|---|
| $SiO_2$ | 62,0 bis 80,0 |
| $Li_2O$ | 7,0 bis 13,0 |
| $Al_2O_3$ | 3,0 bis 12,0 |
| $P_2O_5$ | 0,5 bis 10,0 |
| $Na_2O$ | 0 bis 5,0 |
| $K_2O$ | 0 bis 10,0 |
| $Rb_2O$ | 0 bis 8,0 |
| $Cs_2O$ | 0 bis 8,0 |
| MgO | 0 bis 4,0 |
| CaO | 0 bis 4,0 |
| SrO | 0 bis 10,0 |
| ZnO | 0 bis 6,0 |
| $B_2O_3$ | 0 bis 8,0 |
| $Y_2O_3$ | 0 bis 8,0 |
| $La_2O_3$ | 0 bis 8,0 |
| $Ga_2O_3$ | 0 bis 5,0 |
| $In_2O_3$ | 0 bis 5,0 |
| $TiO_2$ | 0 bis 5,0 |
| $ZrO_2$ | 0 bis 12,0 |
| $GeO_2$ | 0 bis 5,0 |
| $SnO_2$ | 0 bis 5,0 |
| $CeO_2$ | 0 bis 5,0 |
| $V_2O_5$ | 0 bis 2,0 |
| $Nb_2O_5$ | 0 bis 10, 0 |
| $Ta_2O_5$ | 0 bis 10, 0 |
| $MoO_3$ | 0 bis 3,0 |
| $WO_3$ | 0 bis 3,0 |
| Fluor | 0 bis 5,0. |

Manche der vorstehend genannten Komponenten können als Färbemittel und/oder Fluoreszenzmittel dienen.

Die erfindungsgemäße Glaskeramik kann darüber hinaus noch weitere Färbemittel und/oder Fluoreszenzmittel enthalten. Diese können z.B. aus $Bi_2O_3$ oder $Bi_2O_5$ und insbesondere aus weiteren anorganischen Pigmenten und/oder Oxiden von d- und f-Elementen, wie z.B. den Oxiden von Mn, Fe, Co, Pr, Nd, Tb, Er, Dy, Eu und Yb, ausgewählt sein. Mithilfe dieser Färbemittel und Fluoreszenzmittel ist eine einfache Einfärbung der Glaskeramik möglich, um die gewünschten optischen Eigenschaften insbesondere von natürlichem Zahnmaterial zu imitieren.

In einer bevorzugten Ausführungsform der Glaskeramik liegt das molare Verhältnis von $SiO_2$ zu $Li_2O$ im Bereich von 2,5 bis 4,0, bevorzugt im Bereich von 2,8 bis 3,8 und besonders bevorzugt im Bereich von 3,0 bis 3,6.

Typischerweise enthält die erfindungsgemäße Glaskeramik neben der Hauptkristallphase Lithiumdisilikat allenfalls geringe Anteile an Nebenkristallphasen. Vorzugsweise enthält die Glaskeramik weniger als 12 Gew.-%, insbesondere weniger als 10 Gew.-%, bevorzugt weniger als 8 Gew.-% und besonders bevorzugt weniger als 5 Gew.-% an Nebenkristallphasen. Beispiele für solche Nebenkristallphasen sind Lithiummetasilikat-Kristalle, Lithiumphosphat-Kristallen, $SiO_2$-Kristalle wie Quarz-Kristalle oder Cristobalit-Kristalle, $SiO_2$-Mischkristalle wie Quarz-Mischkristalle, Cristobalit-Mischkristalle oder Lithiumaluminiumsilikat-Kristalle, oder $ZrO_2$-Kristalle. Es ist besonders bevorzugt, dass die Glaskeramik weniger als 10 Gew.-%, insbesondere weniger als 7 Gew.-%, bevorzugt weniger als 5 Gew.-%, besonders bevorzugt weniger als 3 Gew.-% und ganz besonders bevorzugt weniger als 1 Gew.-% an Quarz-Kristallen und/oder Quarz-Mischkristallen enthält. Es ist außerdem besonders bevorzugt, dass die erfindungsgemäße Glaskeramik im Wesentlichen frei von Cristobalit ist.

Die Art und insbesondere die Menge der gebildeten Kristallphasen können durch die Zusammensetzung des Ausgangsglases sowie die Wärmebehandlung gesteuert werden, die zur Herstellung der Glaskeramik aus dem Ausgangsglas angewendet wird. Die Beispiele veranschaulichen dies anhand der Variation der Zusammensetzung des Ausgangsglases und der angewendeten Wärmebehandlung.

Die Glaskeramik weist eine biaxiale Bruchfestigkeit $\sigma_B$ von vorzugsweise mindestens 200 MPa und besonders bevorzugt 250 bis 600 MPa auf. Die biaxiale Bruchfestigkeit wurde gemäß ISO 6872 (2008) (Kolben-auf-drei-Kugeln-Prüfung) bestimmt.

Die erfindungsgemäße Glaskeramik weist einen thermischen Ausdehnungskoeffizienten WAK (gemessen im Bereich von 100 bis 500°C) von bevorzugt 8 bis $13 \cdot 10^{-6}$ $K^{-1}$ auf. Der WAK wird gemäß ISO 6872 (2015) bestimmt. Eine Einstellung des Wärmeausdehnungskoeffizienten auf einen gewünschten Wert erfolgt insbesondere durch die Art und Menge der in der Glaskeramik vorhandenen Kristallphasen sowie die chemische Zusammensetzung der Glaskeramik.

Die Transluzenz der Glaskeramik wurde in Form des Kontrastwerts (CR-Wert) gemäß British Standard BS 5612 bestimmt, und dieser Kontrastwert betrug vorzugsweise 30 bis 90 und bevorzugt 40 bis 85.

Die Erfindung betrifft ebenfalls verschiedene Vorstufen mit entsprechender Zusammensetzung, aus denen die erfindungsgemäße Lithiumsilikat-Glaskeramik durch Wärmebehandlung hergestellt werden kann. Diese Vorstufen sind ein entsprechend zusammengesetztes Ausgangsglas und ein entsprechend zusammengesetztes Ausgangsglas mit Keimen. Die Bezeichnung "entsprechende Zusammensetzung" bedeutet, dass diese Vorstufen die gleichen Komponenten in den gleichen Mengen wie die Glaskeramik enthalten, wobei die Komponenten mit Ausnahme von Fluor als Oxide berechnet werden, so wie es bei Gläsern und Glaskeramiken üblich ist.

Die Erfindung betrifft daher ebenfalls ein Ausgangsglas, das die Komponenten der erfindungsgemäßen Lithiumsilikat-Glaskeramik enthält.

Das erfindungsgemäße Ausgangsglas enthält daher insbesondere geeignete Mengen an $SiO_2$ und $Li_2O$, die zur Ausbildung der erfindungsgemäßen Glaskeramik mit Lithiumdisilikat als Hauptkristallphase erforderlich sind. Weiter kann das Ausgangsglas auch noch andere Komponenten enthalten, wie sie oben für die erfindungsgemäße Lithiumsilikat-Glaskeramik angegeben sind. Es sind alle solche Ausführungsformen für die Komponenten des Ausgangsglases bevorzugt, die auch für die Komponenten der erfindungsgemäßen Lithiumsilikat-Glaskeramik als bevorzugt angegeben sind.

Die Erfindung betrifft ebenfalls ein solches Ausgangsglas, das Keime für die Bildung von Lithiumdisilikatkristallen enthält.

Die erfindungsgemäße Lithiumsilikat-Glaskeramik und das erfindungsgemäße Ausgangsglas liegen insbesondere in Form von Pulvern, Granulaten oder Rohlingen in beliebiger Form und Größe, z.B. monolithischen Rohlingen, wie Plättchen, Quadern oder Zylindern, oder Pulverpresslingen, in ungesinterter, teilgesinterter oder dichtgesinterter Form, vor. In diesen Formen können sie einfach weiterverarbeitet werden. Sie können aber auch in Form von dentalen Restaurationen, wie Inlays, Onlays, Kronen, Veneers, Schalen oder Abutments, vorliegen.

Zur Herstellung des Ausgangsglases wird insbesondere so vorgegangen, dass eine Mischung von geeigneten Ausgangsmaterialien, wie z.B. Carbonaten, Oxiden, Phosphaten und Fluoriden, bei Temperaturen von insbesondere 1300 bis 1600°C für 2 bis 10 h erschmolzen wird. Zur Erzielung einer besonders hohen Homogenität wird die erhaltene Glasschmelze in Wasser gegossen, um ein Glasgranulat zu bilden, und das erhaltene Granulat wird dann erneut aufgeschmolzen.

Die Schmelze kann dann in Formen gegossen werden, um Rohlinge des Ausgangsglases, sogenannte Massivglasrohlinge oder monolithische Rohlinge, zu erzeugen.

Es ist ebenfalls möglich, die Schmelze erneut in Wasser zu geben, um ein Granulat herzustellen. Dieses Granulat kann nach Mahlen und gegebenenfalls Zugabe weiterer Komponenten, wie Färbe- und Fluoreszenzmitteln, zu einem Rohling, einem sogenannten Pulverpressling, gepresst werden.

Schließlich kann das Ausgangsglas nach Granulierung auch zu einem Pulver verarbeitet werden.

Anschließend wird das Ausgangsglas, z.B. in Form eines Massivglasrohlings, eines Pulverpresslings oder in Form eines Pulvers, mindestens einer Wärmebehandlung unterzogen. Es ist bevorzugt, dass zunächst eine erste Wärmebehandlung durchgeführt wird, um das erfindungsgemäße Ausgangsglas mit Keimen für die Bildung von Lithiumdisilikatkristallen herzustellen. Das Ausgangsglas mit Keimen wird dann üblicherweise mindestens einer weiteren Temperaturbehandlung bei einer höheren Temperatur unterworfen, um Kristallisation von Lithiumdisilikat zu bewirken und die erfindungsgemäße Lithiumsilikat-Glaskeramik herzustellen.

Die Erfindung betrifft somit ebenfalls ein Verfahren zur Herstellung der erfindungsgemäßen Lithiumsilikat-Glaskeramik, bei dem das Ausgangsglas oder das Ausgangsglas mit Keimen mindestens einer Wärmebehandlung bei einer Temperatur von 400 bis 1000°C für eine Dauer von insbesondere 1 bis 120 min, bevorzugt 5 bis 90 min und besonders bevorzugt 10 bis 60 min, unterzogen wird.

Die im erfindungsgemäßen Verfahren durchgeführte mindestens eine Wärmebehandlung kann auch im Rahmen eines Heißpressens oder Aufsinterns des erfindungsgemäßen Ausgangsglases oder des erfindungsgemäßen Ausgangsglases mit Keimen erfolgen.

Es ist bevorzugt, das Ausgangsglas einer Wärmebehandlung bei einer Temperatur von 400 bis 650°C, bevorzugt 450 bis 600°C und besonders bevorzugt 480 bis 580°C, für eine Dauer von insbesondere 1 bis 240 min, bevorzugt 5 bis 120 min und besonders bevorzugt 10 bis 60 min, zu unterwerfen, um das Ausgangsglas mit Keimen für die Kristallisation von Lithiumdisilikat zu erzeugen.

Es ist weiter bevorzugt, das Ausgangsglas mit Keimen einer Wärmebehandlung bei einer Temperatur von 700 bis 1000°C, bevorzugt 750 bis 950°C und besonders bevorzugt 800 bis 900°C, für eine Dauer von insbesondere 1 bis 120 min, bevorzugt 2 bis 90 min, besonders bevorzugt 5 bis 60 min und ganz besonders bevorzugt 10 bis 30 min, zu unterwerfen, um die Lithiumsilikat-Glaskeramik herzustellen. Die geeigneten Bedingungen für eine gegebene Glaskeramik können beispielsweise bestimmt werden, indem Röntgenbeugungsanalysen bei unterschiedlichen Temperaturen durchgeführt werden.

In einer bevorzugten Ausführungsform umfasst somit das erfindungsgemäße Verfahren zur Herstellung der Lithiumsilikat-Glaskeramik, dass

(a) das Ausgangsglas einer Wärmebehandlung bei einer Temperatur von 400 bis 650°C, bevorzugt 450 bis 600°C und besonders bevorzugt 480 bis 580°C, für eine Dauer von insbesondere 1 bis 240 min, bevorzugt 5 bis 120 min und besonders bevorzugt 10 bis 60 min, unterworfen wird, um Ausgangsglas mit Keimen zu bilden, und

(b) das Ausgangsglas mit Keimen einer Wärmebehandlung bei einer Temperatur von 700 bis 1000°C, bevorzugt 750 bis 950°C und besonders bevorzugt 800 bis 900°C, für eine Dauer von insbesondere 1 bis 120 min, bevorzugt 2 bis 90 min, besonders bevorzugt 5 bis 60 min und ganz besonders bevorzugt 10 bis 30 min, unterworfen wird, um die Glaskeramik zu bilden.

Aufgrund der vorstehend geschilderten Eigenschaften der erfindungsgemäßen Glaskeramiken und der erfindungsgemäßen Gläser eignen sich diese insbesondere zum Einsatz in der Zahnheilkunde. Gegenstand der Erfindung ist daher auch die Verwendung der erfindungsgemäßen Glaskeramiken oder der erfindungsgemäßen Gläser als Dentalmaterial und insbesondere zur Herstellung dentaler Restaurationen oder als Beschichtungsmaterial für dentale Restaurationen.

Insbesondere können aus den erfindungsgemäßen Glaskeramiken und den erfindungsgemäßen Gläsern dentale Restaurationen, wie Brücken, Inlays, Onlays, Veneers, Abutments, Teilkronen, Kronen oder Schalen, hergestellt werden. Die Erfindung betrifft daher auch die Verwendung der erfindungsgemäßen Glaskeramiken oder der erfindungsgemäßen Gläser zur Herstellung dentaler Restaurationen. Dabei ist es bevorzugt, dass der Glaskeramik oder dem Glas durch Verpressen oder maschinelle Bearbeitung die Form der gewünschten dentalen Restauration gegeben wird.

Die Erfindung betrifft auch ein Verfahren zur Herstellung von dentalen Restaurationen, bei dem den erfindungsgemäßen Glaskeramiken oder den erfindungsgemäßen Gläsern durch Verpressen oder maschinelle Bearbeitung die Form der gewünschten dentalen Restauration gegeben wird.

Das Verpressen erfolgt üblicherweise unter erhöhtem Druck und erhöhter Temperatur. Es ist bevorzugt, dass das Verpressen bei einer Temperatur von 700 bis 1200°C erfolgt. Weiter ist es bevorzugt, das Verpressen bei einem Druck von 2 bis 10 bar durchzuführen. Beim Verpressen wird durch viskoses Fließen des eingesetzten Materials die gewünschte Formänderung erreicht. Es können für das Verpressen das erfindungsgemäße Ausgangsglas und insbesondere das erfindungsgemäße Ausgangsglas mit Keimen, und die erfindungsgemäße Lithiumsilikat-Glaskeramik verwendet werden. Dabei können die erfindungsgemäßen Gläser und Glaskeramiken insbesondere in Form von Rohlingen in beliebiger Form und Größe, z.B. Massivrohlingen oder Pulverpresslingen, z.B. in ungesinterter, teilgesinterter oder dichtgesinterter Form, eingesetzt werden.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens durchgeführt wird. Für die maschinelle Bearbeitung können das erfindungsgemäße Ausgangsglas, das erfindungsgemäße Ausgangsglas mit Keimen und die erfindungsgemäße Lithiumsilikat-Glaskeramik verwendet werden. Dabei können die erfindungsgemäßen Gläser und Glaskeramiken insbesondere in Form von Rohlingen, z.B. Massivrohlingen oder Pulverpresslingen, z.B. in ungesinterter, teilgesinterter oder dichtgesinterter Form, eingesetzt werden. Für die maschinelle Bearbeitung wird bevorzugt die erfindungsgemäße Lithiumsilikat-Glaskeramik verwendet.

Es hat sich überraschend gezeigt, dass sich die erfindungsgemäßen Lithiumsilikat-Glaskeramiken bei gleicher Krafteinwirkung schneller als bekannte Lithiumsilikat-Glaskeramiken bearbeiten lassen. Zur Beschreibung dieser Eigenschaft kann insbesondere die Abtragrate an Probenkörpern der Glaskeramiken bestimmt werden. Hierzu werden von den Probenkörpern Plättchen abgesägt und gewogen. Anschließend werden die Plättchen auf einen Halter geklebt und unter Wasserkühlung mit einer automatischen Schleifmaschine, wie sie beispielsweise von der Fa. Struers erhältlich ist, mittels einer Diamantschleifscheibe, beispielsweise mit einer Körnung von 20 $\mu$m, beschliffen. Der Druck der Schleifmaschine wird dabei so gewählt, dass auf jedes Plättchen dieselbe Kraft, beispielsweise 15 N, einwirkt. Nachdem die Plättchen 1 min lang beschliffen wurden, werden diese getrocknet und erneut gewogenen. Die Abtragrate wird dann gemäß der folgenden Formel berechnet:

$$\text{Abtragrate } [\text{Gew.-\% } \cdot \text{min}^{-1}] = 100 \times (1 - (m_{\text{beschliffen}} : m_{\text{unbeschliffen}}))$$

Nachdem die Glaskeramik die Form der gewünschten dentalen Restauration erhalten hat, kann diese einer weiteren Wärmebehandlung unterworfen werden, um weiteres Wachstum von Lithiumdisilikat-Kristallen zu bewirken. Beispielsweise wird dazu die Glaskeramik einer Wärmebehandlung bei einer Temperatur von 700 bis 1000°C, bevorzugt 750 bis 950°C und besonders bevorzugt 800 bis 900°C, insbesondere für eine Dauer von 1 bis 90 Minuten, bevorzugt 2 bis 60 Minuten, besonders bevorzugt 5 bis 30 Minuten und ganz besonders bevorzugt 10 bis 15 Minuten, unterworfen.

Es hat sich allerdings überraschend gezeigt, dass die maschinell bearbeitbare Lithiumsilikat-Glaskeramik mit Lithiumdisilikat als Hauptkristallphase auch ohne eine weitere Wärmebehandlung nicht nur mechanische Eigenschaften wie etwa ausreichende Festigkeit aufweist, sondern zeigt auch andere Eigenschaften zeigt, die für ein Material für Dentalrestaurationen erforderlich sind. Bevorzugt wird die erfindungsgemäße Glaskeramik daher ohne weitere Wärmebehandlung als Dentalmaterial eingesetzt.

Die erfindungsgemäßen Glaskeramiken und die erfindungsgemäßen Gläser eignen sich allerdings auch als Beschichtungsmaterial von z.B. Keramiken und Glaskeramiken. Die Erfindung ist daher ebenfalls auf die Verwendung der erfindungsgemäßen Gläser oder der erfindungsgemäßen Glaskeramiken zur Beschichtung von Keramiken, Glaskeramiken und insbesondere von dentalen Restaurationen gerichtet.

Die Erfindung betrifft auch ein Verfahren zur Beschichtung von Keramiken, Metallen, Metalllegierungen und Glaskeramiken, bei dem erfindungsgemäße Glaskeramik oder erfindungsgemäßes Glas auf die Keramik oder Glaskeramik aufgebracht und erhöhter Temperatur ausgesetzt wird.

Dies kann insbesondere durch Aufsintern oder durch Fügen eines mittels CAD-CAM hergestellten Überwurfs mit einem geeigneten Glaslot oder Kleber und bevorzugt durch Aufpressen erfolgen. Beim Aufsintern wird die Glaskeramik oder das Glas in üblicher Weise, z.B. als Pulver, auf das zu beschichtende Material, wie Keramik oder Glaskeramik, aufgebracht und anschließend bei erhöhter Temperatur gesintert. Bei dem bevorzugten Aufpressen wird die erfindungsgemäße Glaskeramik oder das erfindungsgemäße Glas, z.B. in Form von Pulverpresslingen oder monolithischen Rohlingen, bei einer erhöhten Temperatur, von z.B. 700 bis 1200°C, und unter Anwendung von Druck, z.B. 2 bis 10 bar, aufgepresst. Hierzu können insbesondere die in der EP 231 773 beschriebenen Verfahren und der dort offenbarte Pressofen eingesetzt werden. Ein geeigneter Ofen ist z.B. der Programat EP 5000 von Ivoclar Vivadent AG, Liechtenstein.

Es ist bevorzugt, dass nach Abschluss des Beschichtungsvorganges eine Glaskeramik mit Lithiumsilikat, insbesondere Lithiumdisilikat, als Hauptkristallphase vorliegt, da eine solche Glaskeramik über besonders gute Eigenschaften verfügt.

Die Erfindung wird im Folgenden anhand von sie nicht-beschränkenden Beispielen näher erläutert.

Beispiele

Es wurden insgesamt 77 erfindungsgemäße Gläser und ein Vergleichsbeispiel mit den in der Tabelle I angegebenen Zusammensetzungen hergestellt. Die Gläser wurden gemäß Tabelle II zu Glaskeramiken kristallisiert. Dabei bedeuten

$T_g$: Glasübergangstemperatur, bestimmt mittels DSC

$T_s$ und $t_s$: angewendete Temperatur und Zeit zum Erschmelzen

$T_1$ und $t_1$: angewendete Temperatur und Zeit für 1. Wärmebehandlung

$T_2$ und $t_2$: angewendete Temperatur und Zeit für 2. Wärmebehandlung

Es wurden zunächst Ausgangsgläser mit den in der Tabelle I angegebenen Zusammensetzungen im 100 bis 200 g Maßstab aus üblichen Rohstoffen bei der Temperatur $T_s$ für die Dauer $t_s$ erschmolzen, wobei das Erschmelzen sehr gut ohne Bildung von Blasen oder Schlieren möglich war. Durch Eingießen dieser Ausgangsgläser in Wasser wurden Glasgranulate hergestellt.

In den Beispielen 1-70 wurden die Glasgranulate zur Homogenisierung ein zweites Mal bei der Temperatur $T_s$ für die Dauer $t_s$ geschmolzen wurden. Die erhaltenen Schmelzen des Ausgangsglases wurden in eine Graphitform gegossen, um Massivglasblöcke zu erzeugen. Eine erste Wärmebehandlung der so erhaltenen Glasmonolithe bei der Temperatur $T_1$ für die Dauer $t_1$ führte zur Bildung von Gläsern mit Keimen. Diese keimhaltigen Gläser kristallisierten durch weitere Wärmebehandlung bei der Temperatur $T_2$ für die Dauer $t_2$ zu Glaskeramiken.

In den Beispielen 71-77 wurden die Glasgranulate bei 150°C für 1 h im Trockenschrank getrocknet, in einer mit Zirkonoxid ausgekleideten Mühle auf <90 $\mu$m aufgemahlen und abgesiebt. Die so erhaltenen Glaspulver wurden mittels einer uniaxialen Presse bei 10 bar zu quaderförmigen Rohlingen verpresst. Die Rohlinge wurden unter Vakuum mit einer Aufheizgeschwindigkeit von 10 K/min zunächst auf die Temperatur $T_1$ aufgeheizt und für die Dauer $t_1$ bei dieser Temperatur gehalten. Anschließend wurden die Rohlinge mit einer Aufheizgeschwindigkeit von 10 K/min weiter auf die Temperatur $T_2$ aufgeheizt und für die Dauer $t_2$ bei dieser Temperatur gehalten. Schließlich wurden die Rohlinge auf Raumtemperatur abgekühlt.

Wie durch Röntgenbeugungsuntersuchungen bei Raumtemperatur festgestellt wurde, wurden in allen Fällen Glaskeramiken mit Lithiumdisilikat als Hauptkristallphase erhalten.

Die Mengen der Kristallphasen wurden mittels Röntgenbeugung bestimmt. Dazu wurde mittels Mahlen und Sieben (< 45 $\mu$m) ein Pulver der jeweiligen Glaskeramik hergestellt und mit $Al_2O_3$ (Alfa Aesar, Produkt-Nr. 42571) als internem Standard im Verhältnis von 80 Gew.-% Glaskeramik zu 20 Gew.-% $Al_2O_3$ gemischt. Diese Mischung wurde mit Aceton aufgeschlämmt, um eine möglichst gute Durchmischung zu erreichen. Anschließend wurde die Mischung bei etwa 80°C getrocknet. Danach wurde mittels eines D8 Advance Diffraktometers der Firma Bruker ein Diffraktogramm im Bereich 10 bis 100° $2\theta$ mittels $CuK\alpha$-Strahlung und einer Schrittweite von 0,014° $2\theta$ aufgenommen. Dieses Diffraktogramm wurde dann mit der Software TOPAS 5.0 der Firma Bruker nach der Rietveld-Methode ausgewertet.

Die mittlere Länge und das mittlere Aspektverhältnis der Lithiumdisilikatkristalle wurden anhand von SEM-Aufnahmen bestimmt. Dazu wurde eine Oberfläche der jeweiligen Glaskeramik poliert (< 0,5 $\mu$m), für mindestens 30 s mit 40%igem HF-Dampf geätzt und anschließend mit einer Au-Pd Schicht besputtert. Von den so behandelten Oberflächen wurden SEM-Bilder mit einem Rasterelektronenmikroskop des Typs Supra 40VP (Zeiss, Oberkochen, Deutschland) aufgenommen. Die SEM-Aufnahmen wurden zur Verbesserung des Kontrasts zwischen Kristallen und Glasphase mit einem üblichen Bildbearbeitungsprogramm nachbearbeitet. Anschließend wurden die mittlere Länge und das mittlere Aspektverhältnis der Kristalle mit der Bildanalysesoftware Olympus Stream Motion 2.4 (Olympus Corporation, Tokyo, Japan) bestimmt.

Zur Bestimmung der mechanischen Bearbeitbarkeit wurden von den so erhaltenen Glaskeramikblöcken je zwei Plättchen mit einer Fläche von 170 mm$^2$ $\pm$ 10 mm$^2$ (ca. 12.5 mm x 13.8 mm) und einer Dicke von 4,0 $\pm$ 0,5 mm abgesägt und auf einer Präzisionswaage gewogen. Anschließend wurden die Plättchen auf einen Halter geklebt und unter Wasserkühlung mit einer automatischen Schleifmaschine (LaboForche-100, Fa. Struers) mittels einer Diamantschleifscheibe mit einer Körnung von 20 $\mu$m beschliffen. Der Druck der Schleifmaschine wurde dabei so gewählt, dass auf jedes Plättchen eine Kraft von 15 N einwirkte. Der Drehteller, auf dem die Diamantschleifscheibe befestigt war, und der Kopf der Schleifmaschine, auf dem die Halter mit den Proben befestigt war, wiesen die gleiche Drehrichtung auf. Die Geschwindigkeit des Drehtellers betrug 300 U min$^{-1}$. Die Plättchen wurden 1 min lang beschliffen und anschließend getrocknet und erneut gewogenen. Die Abtragrate wurde gemäß der folgenden Formel berechnet:

$$\text{Abtragrate } [\text{Gew.-\% } \cdot \text{min}^{-1}] = 100 \times (1 - (m_{\text{beschliffen}} : m_{\text{unbeschliffen}}))$$

Wie aus Tabelle II ersichtlich ist, waren die Abtragraten bei den erfindungsgemäßen Beispielen durchgehend höher als im Vergleichsbeispiel. Dies zeigt, dass sich die erfindungsgemäßen Lithiumsilikat-Glaskeramiken bei gleicher Krafteinwirkung schneller als bekannte Lithiumdisilikat-Glaskeramiken bearbeiten lassen.

Tabelle I

| Beispiel | 1 | 2 | 3 | 4 | 5 | 6 | 7 | 8 | 9 | 10 |
|---|---|---|---|---|---|---|---|---|---|---|
| Zusammensetzung | Gew.-% | Gew.-% | Gew.-% | Gew.-% | Gew.-% | Gew.-% | Gew.-% | Gew.-% | Gew.-% | Gew.-% |
| $SiO_2$ | 68,8 | 67,8 | 67,0 | 67,6 | 66,4 | 66,6 | 64,2 | 69,7 | 70,1 | 66,2 |
| $Li_2O$ | 9,9 | 9,7 | 9,5 | 9,6 | 9,4 | 9,5 | 9,1 | 11,5 | 11,7 | 11,0 |
| $Na_2O$ | – | – | – | – | – | – | – | – | – | 3,0 |
| $K_2O$ | 5,8 | 5,7 | 5,6 | 5,7 | 5,5 | 5,6 | 5,4 | 3,3 | 2,3 | – |
| $Rb_2O$ | – | – | – | – | – | – | – | – | – | 5,0 |
| $Cs_2O$ | – | – | – | – | – | – | – | – | – | – |
| $MgO$ | – | 1,4 | 2,7 | – | – | – | – | – | – | – |
| $CaO$ | – | – | – | 1,9 | 3,8 | 3,4 | – | – | – | – |
| $SrO$ | – | – | – | – | – | – | 6,7 | – | – | – |
| $ZnO$ | – | – | – | – | – | – | – | – | – | – |
| $Al_2O_3$ | 5,0 | 5,0 | 4,9 | 4,9 | 4,8 | 4,8 | 4,7 | 4,6 | 4,7 | 4,0 |
| $Y_2O_3$ | – | – | – | – | – | – | – | 2,0 | 2,1 | – |
| $La_2O_3$ | – | – | – | – | – | – | – | | | – |
| $ZrO_2$ | 8,0 | 7,9 | 7,8 | 7,8 | 7,7 | 7,7 | 7,5 | 6,1 | 6,2 | 8,0 |
| $CeO_2$ | – | – | – | – | – | – | – | – | – | – |
| $P_2O_5$ | 2,5 | 2,5 | 2,5 | 2,5 | 2,4 | 2,4 | 2,4 | 2,8 | 2,9 | 2,8 |
| $V_2O_5$ | – | – | – | – | – | – | – | – | – | – |
| $Nb_2O_5$ | – | – | – | – | – | – | – | – | – | – |
| $Ta_2O_5$ | – | – | – | – | – | – | – | – | – | – |
| $Gd_2O_3$ | – | – | – | – | – | – | – | – | – | – |
| $Tb_4O_7$ | – | – | – | – | – | – | – | – | – | – |
| $Er_2O_3$ | – | – | – | – | – | – | – | – | – | – |
| $F$ | – | – | – | – | – | – | – | – | – | – |
| $\sum$ | 100,0 | 100,0 | 100,0 | 100,0 | 100,0 | 100,0 | 100,0 | 100,0 | 100,0 | 100,0 |

Tabelle I

| Beispiel | 11 | 12 | 13 | 14 | 15 | 16 | 17 | 18 | 19 | 20 |
|---|---|---|---|---|---|---|---|---|---|---|
| Zusammen setzung | Gew.-% | Gew.-% | Gew.-% | Gew.-% | Gew.-% | Gew.-% | Gew.-% | Gew.-% | Gew.-% | Gew.-% |
| $SiO_2$ | 65,4 | 68,8 | 68,8 | 68,7 | 68,9 | 68,9 | 68,9 | 68,9 | 67,5 | 67,7 |
| $Li_2O$ | 10,8 | 11,4 | 9,8 | 9,8 | 9,8 | 9,8 | 9,8 | 9,8 | 11,2 | 11,2 |
| $Na_2O$ | 3,00 | 2,0 | – | – | – | – | – | – | – | – |
| $K_2O$ | – | 3,0 | 5,8 | 5,8 | 5,8 | 5,8 | 5,8 | 5,8 | 5,8 | 5,8 |
| $Rb_2O$ | – | – | – | – | – | – | – | – | – | – |
| $Cs_2O$ | 6,0 | – | – | – | – | – | – | – | – | – |
| $MgO$ | – | – | – | – | – | – | – | – | – | – |
| $CaO$ | – | – | – | – | – | – | – | – | – | – |
| $SrO$ | – | – | – | – | 2,0 | 4,0 | – | – | – | – |
| $ZnO$ | – | – | – | – | – | – | – | – | – | – |
| $Al_2O_3$ | 4,0 | 4,0 | 5,0 | 5,0 | 5,0 | 5,0 | 5,0 | 5,0 | 5,0 | 4,5 |
| $Y_2O_3$ | – | – | – | – | – | – | – | – | – | – |
| $La_2O_3$ | – | – | – | – | – | – | – | – | – | – |
| $ZrO_2$ | 8,0 | 8,0 | 8,0 | 8,0 | 6,0 | 4,0 | 6,0 | 4,0 | 8,0 | 8,0 |
| $CeO_2$ | – | – | – | – | – | – | – | – | – | – |
| $P_2O_5$ | 2,8 | 2,8 | 2,6 | 2,7 | 2,5 | 2,5 | 2,5 | 2,5 | 2,5 | 2,8 |
| $V_2O_5$ | – | – | – | – | – | – | – | – | – | – |
| $Nb_2O_5$ | – | – | – | – | – | – | – | – | – | – |
| $Ta_2O_5$ | – | – | – | – | – | – | 2,0 | 4,0 | – | – |
| $Gd_2O_3$ | – | – | – | – | – | – | – | – | – | – |
| $Tb_4O_7$ | – | – | – | – | – | – | – | – | – | – |
| $Er_2O_3$ | – | – | – | – | – | – | – | – | – | – |
| $F$ | – | – | – | – | – | – | – | – | – | – |
| $\sum$ | 100,0 | 100,0 | 100,0 | 100,0 | 100,0 | 100,0 | 100,0 | 100,0 | 100,0 | 100,0 |

## Tabelle I

| Beispiel | 21 | 22 | 23 | 24 | 25 | 26 | 27 | 28 | 29 | 30 |
|---|---|---|---|---|---|---|---|---|---|---|
| Zusammen setzung | Gew.-% | Gew.-% | Gew.-% | Gew.-% | Gew.-% | Gew.-% | Gew.-% | Gew.-% | Gew.-% | Gew.-% |
| $SiO_2$ | 68,1 | 68,1 | 68,1 | 68,1 | 68,4 | 68,5 | 68,8 | 69,4 | 69,0 | 69,2 |
| $Li_2O$ | 11,3 | 11,3 | 11,3 | 11,3 | 11,3 | 11,4 | 11,4 | 10,8 | 10,7 | 10,7 |
| $Na_2O$ | – | – | – | – | – | – | – | – | – | – |
| $K_2O$ | 5,8 | 5,8 | 5,8 | 5,8 | 5,5 | 5,3 | 5,0 | 5,0 | 5,5 | 5,5 |
| $Rb_2O$ | – | – | – | – | – | – | – | – | – | – |
| $Cs_2O$ | – | – | – | – | – | – | – | – | – | – |
| $MgO$ | – | – | – | – | – | – | – | – | – | – |
| $CaO$ | – | – | – | – | – | – | – | – | – | – |
| $SrO$ | – | – | – | – | – | – | – | – | – | – |
| $ZnO$ | – | – | – | – | – | – | – | – | – | – |
| $Al_2O_3$ | 4,0 | 4,0 | 4,0 | 4,0 | 4,0 | 4,0 | 4,0 | 4,0 | 4,0 | 4,0 |
| $Y_2O_3$ | – | – | – | – | – | – | – | – | – | – |
| $La_2O_3$ | – | 0,5 | 1,0 | – | – | – | – | – | – | – |
| $ZrO_2$ | 8,0 | 7,5 | 7,0 | 8,0 | 8,0 | 8,0 | 8,0 | 8,0 | 8,0 | 8,0 |
| $CeO_2$ | – | – | – | – | – | – | – | – | – | – |
| $P_2O_5$ | 2,8 | 2,8 | 2,8 | 2,8 | 2,8 | 2,8 | 2,8 | 2,8 | 2,8 | 2,6 |
| $V_2O_5$ | – | – | – | – | – | – | – | – | – | – |
| $Nb_2O_5$ | – | – | – | – | – | – | – | – | – | – |
| $Ta_2O_5$ | – | – | – | – | – | – | – | – | – | – |
| $Gd_2O_3$ | – | – | – | – | – | – | – | – | – | – |
| $Tb_4O_7$ | – | – | – | – | – | – | – | – | – | – |
| $Er_2O_3$ | – | – | – | – | – | – | – | – | – | – |
| $F$ | – | – | – | – | – | – | – | – | – | – |
| $\Sigma$ | 100,0 | 100,0 | 100,0 | 100,0 | 100,0 | 100,0 | 100,0 | 100,0 | 100,0 | 100,0 |

EP 4 382 495 A1

## Tabelle I

| Beispiel | 31 | 32 | 33 | 34 | 35 | 36 | 37 | 38 | 39 | 40 |
|---|---|---|---|---|---|---|---|---|---|---|
| Zusammen setzung | Gew.-% | Gew.-% | Gew.-% | Gew.-% | Gew.-% | Gew.-% | Gew.-% | Gew.-% | Gew.-% | Gew.-% |
| $SiO_2$ | 69,3 | 70,0 | 69,8 | 70,0 | 70,3 | 70,5 | 70,3 | 70,6 | 71,1 | 70,8 |
| $Li_2O$ | 10,8 | 10,2 | 10,2 | 10,3 | 10,3 | 9,7 | 9,7 | 9,8 | 9,8 | 10,4 |
| $Na_2O$ | – | – | – | – | – | – | – | – | – | – |
| $K_2O$ | 5,5 | 5,0 | 5,2 | 5,2 | 5,2 | 5,0 | 5,2 | 5,2 | 5,2 | 5,0 |
| $Rb_2O$ | – | – | – | – | – | – | – | – | – | – |
| $Cs_2O$ | – | – | – | – | – | – | – | – | – | – |
| $MgO$ | – | – | – | – | – | – | – | – | – | – |
| $CaO$ | – | – | – | – | – | – | – | – | – | – |
| $SrO$ | – | – | – | – | – | – | – | – | – | – |
| $ZnO$ | – | – | – | – | – | – | – | – | – | – |
| $Al_2O_3$ | 4,0 | 4,0 | 4,0 | 4,0 | 4,0 | 4,0 | 4,0 | 4,0 | 4,0 | 4,0 |
| $Y_2O_3$ | – | – | – | – | – | – | – | – | – | – |
| $La_2O_3$ | – | – | – | – | – | – | – | – | – | – |
| $ZrO_2$ | 8,0 | 8,0 | 8,0 | 8,0 | 8,0 | 8,0 | 8,0 | 8,0 | 8,0 | 7,0 |
| $CeO_2$ | – | – | – | – | – | – | – | – | – | – |
| $P_2O_5$ | 2,4 | 2,8 | 2,8 | 2,5 | 2,2 | 2,8 | 2,8 | 2,4 | 1,9 | 2,8 |
| $V_2O_5$ | – | – | – | – | – | – | – | – | – | – |
| $Nb_2O_5$ | – | – | – | – | – | – | – | – | – | – |
| $Ta_2O_5$ | – | – | – | – | – | – | – | – | – | – |
| $Gd_2O_3$ | – | – | – | – | – | – | – | – | – | – |
| $Tb_4O_7$ | – | – | – | – | – | – | – | – | – | – |
| $Er_2O_3$ | – | – | – | – | – | – | – | – | – | – |
| $F$ | – | – | – | – | – | – | – | – | – | – |
| $\sum$ | 100,0 | 100,0 | 100,0 | 100,0 | 100,0 | 100,0 | 100,0 | 100,0 | 100,0 | 100,0 |

Tabelle I

| Beispiel | 41 | 42 | 43 | 44 | 45 | 46 | 47 | 48 | 49 | 50 |
|---|---|---|---|---|---|---|---|---|---|---|
| Zusammen setzung | Gew.-% | Gew.-% | Gew.-% | Gew.-% | Gew.-% | Gew.-% | Gew.-% | Gew.-% | Gew.-% | Gew.-% |
| $SiO_2$ | 71,7 | 71,3 | 72,6 | 68,1 | 70,0 | 70,5 | 68,1 | 68,1 | 68,1 | 68,9 |
| $Li_2O$ | 10,5 | 10,4 | 10,6 | 11,3 | 10,2 | 9,7 | 11,3 | 11,3 | 11,3 | 9,8 |
| $Na_2O$ | – | – | – | – | – | – | – | – | – | – |
| $K_2O$ | 5,0 | 5,5 | 5,0 | 5,8 | 5,0 | 5,0 | 5,1 | 5,1 | 5,1 | 5,8 |
| $Rb_2O$ | – | – | – | – | – | – | – | – | – | – |
| $Cs_2O$ | – | – | – | – | – | – | – | – | – | – |
| $MgO$ | – | – | – | – | – | – | – | – | – | – |
| $CaO$ | – | – | – | – | – | – | – | – | 0,7 | – |
| $SrO$ | – | – | – | – | – | – | 0,7 | 0,7 | – | – |
| $ZnO$ | – | – | – | – | – | – | – | – | – | – |
| $Al_2O_3$ | 4,0 | 4,0 | 4,0 | 4,0 | 4,0 | 4,0 | 4,0 | 4,0 | 4,0 | 5,0 |
| $Y_2O_3$ | – | – | – | 0,5 | 1,0 | 2,0 | 1,0 | – | – | – |
| $La_2O_3$ | – | – | – | – | – | – | – | 1,0 | 1,0 | – |
| $ZrO_2$ | 6,0 | 6,0 | 5,0 | 7,5 | 7,0 | 6,0 | 7,0 | 7,0 | 7,0 | – |
| $CeO_2$ | – | – | – | – | – | – | – | – | – | – |
| $P_2O_5$ | 2,8 | 2,8 | 2,8 | 2,8 | 2,8 | 2,8 | 2,8 | 2,8 | 2,8 | 2,5 |
| $V_2O_5$ | – | – | – | – | – | – | – | – | – | – |
| $Nb_2O_5$ | – | – | – | – | – | – | – | – | – | 8,0 |
| $Ta_2O_5$ | – | – | – | – | – | – | – | – | – | – |
| $Gd_2O_3$ | – | – | – | – | – | – | – | – | – | – |
| $Tb_4O_7$ | – | – | – | – | – | – | – | – | – | – |
| $Er_2O_3$ | – | – | – | – | – | – | – | – | – | – |
| $F$ | – | – | – | – | – | – | – | – | – | – |
| $\Sigma$ | 100,0 | 100,0 | 100,0 | 100,0 | 100,0 | 100,0 | 100,0 | 100,0 | 100,0 | 100,0 |

EP 4 382 495 A1

Tabelle I

| Beispiel | 51 | 52 | 53 | 54 | 55 | 56 | 57 | 58 | 59 | 60 |
|---|---|---|---|---|---|---|---|---|---|---|
| Zusammensetzung | Gew.-% | Gew.-% | Gew.-% | Gew.-% | Gew.-% | Gew.-% | Gew.-% | Gew.-% | Gew.-% | Gew.-% |
| $SiO_2$ | 68,9 | 72,4 | 70,7 | 70,7 | 71,4 | 73,5 | 71,8 | 71,8 | 72,5 | 72,4 |
| $Li_2O$ | 9,8 | 10,3 | 10,0 | 10,0 | 10,1 | 10,5 | 10,2 | 10,2 | 10,3 | 10,3 |
| $Na_2O$ | – | – | – | – | – | – | – | – | – | – |
| $K_2O$ | 5,8 | 5,8 | 5,8 | 5,8 | 5,0 | 5,8 | 5,8 | 5,8 | 5,0 | 5,8 |
| $Rb_2O$ | – | – | – | – | – | – | – | – | – | – |
| $Cs_2O$ | – | – | – | – | – | – | – | – | – | – |
| $MgO$ | – | – | – | – | – | – | – | – | – | – |
| $CaO$ | – | – | – | – | – | – | – | – | – | – |
| $SrO$ | – | – | – | – | – | – | – | – | – | – |
| $ZnO$ | – | – | – | – | – | – | – | – | – | – |
| $Al_2O_3$ | 5,0 | 5,0 | 5,0 | 5,0 | 5,0 | 5,0 | 5,0 | 5,0 | 5,0 | 5,0 |
| $Y_2O_3$ | – | – | – | – | – | 2,7 | 2,7 | 2,7 | 2,7 | – |
| $La_2O_3$ | – | 4,0 | 4,0 | 4,0 | 4,0 | – | – | – | – | – |
| $ZrO_2$ | – | – | – | – | – | – | – | – | – | – |
| $CeO_2$ | – | – | – | – | – | – | – | – | – | – |
| $P_2O_5$ | 2,5 | 2,5 | 2,5 | 2,5 | 2,5 | 2,5 | 2,5 | 2,5 | 2,5 | 2,5 |
| $V_2O_5$ | – | – | – | – | – | – | – | – | – | – |
| $Nb_2O_5$ | – | – | – | 2,0 | 2,0 | – | – | 2,0 | – | – |
| $Ta_2O_5$ | 8,0 | – | 2,0 | – | – | – | 2,0 | – | 2,0 | – |
| $Gd_2O_3$ | – | – | – | – | – | – | – | – | – | 4,0 |
| $Tb_4O_7$ | – | – | – | – | – | – | – | – | – | – |
| $Er_2O_3$ | – | – | – | – | – | – | – | – | – | – |
| F | – | – | – | – | – | – | – | – | – | – |
| $\Sigma$ | 100,0 | 100,0 | 100,0 | 100,0 | 100,0 | 100,0 | 100,0 | 100,0 | 100,0 | 100,0 |

Tabelle I

| Beispiel | 61 | 62 | 63 | 64 | 65 | 66 | 67 | 68 | 69 | 70 |
|---|---|---|---|---|---|---|---|---|---|---|
| Zusammen setzung | Gew.-% | Gew.-% | Gew.-% | Gew.-% | Gew.-% | Gew.-% | Gew.-% | Gew.-% | Gew.-% | Gew.-% |
| $SiO_2$ | 72,4 | 71,2 | 68,9 | 68,9 | 70,7 | 70,7 | 69,7 | 75,00 | 75,00 | 72,6 |
| $Li_2O$ | 10,3 | 10,1 | 9,8 | 9,8 | 10,0 | 10,0 | 9,9 | 10,7 | 10,7 | 10,2 |
| $Na_2O$ | – | – | – | – | – | – | – | – | – | – |
| $K_2O$ | 7,8 | 5,2 | 5,8 | 5,8 | 5,8 | 5,8 | 6,0 | 5,8 | 5,8 | 5,0 |
| $Rb_2O$ | – | – | – | – | – | – | – | – | – | – |
| $Cs_2O$ | – | – | – | – | – | – | – | – | – | – |
| $MgO$ | – | – | – | – | – | – | – | – | – | – |
| $CaO$ | – | – | – | – | – | – | – | – | – | 1,0 |
| $SrO$ | – | – | – | – | – | – | – | – | – | – |
| $ZnO$ | – | – | – | – | – | – | – | – | – | – |
| $Al_2O_3$ | 3,0 | 7,0 | 5,0 | 4,0 | 5,0 | 5,0 | 6,0 | 5,0 | 5,0 | 4,7 |
| $Y_2O_3$ | – | – | – | – | – | – | – | – | – | – |
| $La_2O_3$ | – | – | – | – | – | – | – | – | – | – |
| $ZrO_2$ | – | – | 4,0 | 4,0 | – | – | – | 1,0 | 1,0 | 1,0 |
| $CeO_2$ | – | – | – | – | – | – | – | – | – | – |
| $P_2O_5$ | 2,5 | 2,5 | 2,5 | 2,5 | 2,5 | 2,5 | 2,4 | 2,5 | 2,5 | 5,0 |
| $V_2O_5$ | – | – | – | – | – | – | – | – | – | – |
| $Nb_2O_5$ | – | – | – | – | – | 2,0 | 2,0 | – | – | – |
| $Ta_2O_5$ | – | – | – | – | 2,0 | – | – | – | – | – |
| $Gd_2O_3$ | 4,0 | 4,0 | 4,0 | 5,0 | 4,0 | 4,0 | 4,0 | – | – | – |
| $Tb_4O_7$ | – | – | – | – | – | – | – | – | – | – |
| $Er_2O_3$ | – | – | – | – | – | – | – | – | – | – |
| $F$ | – | – | – | – | – | – | – | – | – | 0,5 |
| $\sum$ | 100,0 | 100,0 | 100,0 | 100,0 | 100,0 | 100,0 | 100,0 | 100,0 | 100,0 | 100,0 |

16

Tabelle I

| Beispiel | 71 | 72 | 73 | 74 | 75 | 76 | 77 | VGB |
|---|---|---|---|---|---|---|---|---|
| Zusammen setzung | Gew.-% | Gew.-% | Gew.-% | Gew.-% | Gew.-% | Gew.-% | Gew.-% | Gew.-% |
| $SiO_2$ | 69,2 | 70,0 | 70,6 | 68,1 | 68,0 | 67,1 | 65,2 | 71,2 |
| $Li_2O$ | 10,7 | 10,3 | 9,8 | 11,3 | 11,3 | 9,5 | 9,3 | 14,8 |
| $Na_2O$ | – | – | – | – | – | – | – | – |
| $K_2O$ | 5,5 | 5,2 | 5,2 | 5,7 | 5,7 | 5,6 | 5,4 | 4,0 |
| $Rb_2O$ | – | – | – | – | – | – | – | – |
| $Cs_2O$ | – | – | – | – | – | – | – | – |
| $MgO$ | – | – | – | – | – | – | – | – |
| $CaO$ | – | – | – | – | – | – | – | – |
| $SrO$ | – | – | – | – | – | – | – | – |
| $ZnO$ | – | – | – | – | – | 2,7 | 5,4 | – |
| $Al_2O_3$ | 4,0 | 4,0 | 4,0 | 4,0 | 4,0 | 4,9 | 4,7 | 3,3 |
| $Y_2O_3$ | – | – | – | 2,0 | 2,0 | – | – | – |
| $La_2O_3$ | – | – | – | – | – | – | – | – |
| $ZrO_2$ | 8,0 | 8,0 | 8,0 | 6,0 | 6,0 | 7,8 | 7,6 | 0,8 |
| $CeO_2$ | – | – | – | – | – | – | – | 1,9 |
| $P_2O_5$ | 2,6 | 2,5 | 2,4 | 2,9 | 3,0 | 2,4 | 2,4 | 3,2 |
| $V_2O_5$ | – | – | – | – | – | – | – | 0,1 |
| $Nb_2O_5$ | – | – | – | – | – | – | – | – |
| $Ta_2O_5$ | – | – | – | – | – | – | – | – |
| $Gd_2O_3$ | – | – | – | – | – | – | – | – |
| $Tb_4O_7$ | – | – | – | – | – | – | – | 0,5 |
| $Er_2O_3$ | – | – | – | – | – | – | – | 0,2 |
| $F$ | – | – | – | – | – | – | – | – |
| $\sum$ | 100,0 | 100,0 | 100,0 | 100,0 | 100,0 | 100,0 | 100,0 | 100,0 |

Tabelle II

| Beispiel | 1 | 2 | 3 | 4 | 5 | 6 | 7 | 8 | 9 | 10 |
|---|---|---|---|---|---|---|---|---|---|---|
| $T_g$ [°C] | 519 | 502 | 502 | 504 | 507 | 502 | 500 | 501 | 501 | 491 |
| $T_s$ [°C] | 1500 | 1550 | 1550 | 1550 | 1550 | 1550 | 1550 | 1550 | 1550 | 1550 |
| $t_s$ [min] | 60 | 60 | 60 | 60 | 60 | 60 | 60 | 60 | 60 | 60 |
| $T_1$ [°C] | 500 | 520 | 520 | 520 | 520 | 520 | 520 | 500 | 500 | 510 |
| $T_1$ [min] | 10 | 10 | 10 | 10 | 10 | 10 | 10 | 10 | 10 | 40 |
| $T_2$ [°C] | 870 | 880 | 840 | 850 | 850 | 830 | 830 | 810 | 810 | 840 |
| $T_2$ [min] | 10 | 10 | 60 | 10 | 10 | 10 | 10 | 10 | 10 | 10 |
| Hauptkristallphase | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ |
| Phasenanteil [Gew.-%] | | | | | | | | | | |
| Mitt. Länge [µm] | | | | | | | | | | |
| Mitt. Aspektverhältnis | | | | | | | | | | |
| weitere Kristallphasen | $Li_3PO_4$ | $Li_3PO_4$ | $Li_3PO_4$ $Li_2SiO_3$ $LiMgPO_4$ | $Li_3PO_4$ | $Li_3PO_4$ | $Li_3PO_4$ | $Li_3PO_4$ | $Li_3PO_4$ | $Li_3PO_4$ $LiAlSi_4O_{10}$ | $Li_3PO_4$ |
| Abtragrate [Gew.-%·$min^{-1}$] | | | | | | | | | | |
| L* | 92,58 | | | | | | | | | |
| a* | −0,81 | | | | | | | | | |
| b* | 6,20 | | | | | | | | | |
| CR | 92,58 | | | | | | | | | |
| $K_{IC}$ [MPa m$^{0.5}$] | 1,68 ±0,17 | | | | | | | | | |
| $\sigma_B$ [MPa] | 452 ±71 | | | | | | | | | |

EP 4 382 495 A1

Tabelle II

| Beispiel | 11 | 12 | 13 | 14 | 15 | 16 | 17 | 18 | 19 | 20 |
|---|---|---|---|---|---|---|---|---|---|---|
| $T_g$ [°C] | 495 | 491 | 522 | 522 | 502 | 482 | 513 | 505 | 506 | 505 |
| $T_S$ [°C] | 1600 | 1600 | 1500 | 1500 | 1500 | 1500 | 1500 | 1500 | 1500 | 1500 |
| $t_S$ [min] | 60 | 60 | 60 | 60 | 60 | 60 | 60 | 60 | 60 | 60 |
| $T_1$ [°C] | 520 | 510 | 500 | 500 | 500 | 500 | 500 | 500 | 500 | 500 |
| $T_1$ [min] | 10 | 10 | 10 | 10 | 10 | 10 | 10 | 10 | 10 | 10 |
| $T_2$ [°C] | 840 | 820 | 870 | 870 | 850 | 820 | 860 | 860 | 870 | 870 |
| $T_2$ [min] | 10 | 10 | 30 | 30 | 30 | 30 | 30 | 30 | 10 | 30 |
| Hauptkris-tallphase | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ |
| Phasenanteil [Gew.-%] | 23,3 | 29,9 | | | | | | | | |
| Mitt. Länge [µm] | 0,41 | 0,19 | | | | | | | | |
| Mitt. Aspekt-verhältnis | 2,44 | 1,97 | | | | | | | | |
| weitere Kris-tallphasen | $Li_3PO_4$ | $Li_3PO_4$ | $Li_3PO_4$ | $Li_3PO_4$ $Li_2SiO_3$ | $Li_3PO_4$ | $Li_3PO_4$ | $Li_3PO_4$ $Li_2SiO_3$ | $Li_3PO_4$ $Li_2SiO_3$ | $Li_3PO_4$ | $Li_3PO_4$ |
| Abtragrate [Gew.-%·min$^{-1}$] | 15,95 | 15,41 | | | | | | | | |
| L* | | | 92,83 | | | | | | | |
| a* | | | -0,82 | | | | | | | |
| b* | | | 5,89 | | | | | | | |
| CR | | | 55,64 | | | | | | | |
| $K_{IC}$ [MPa m$^{0.5}$] | | | | | | | | | | 1,98 ±0,21 |
| $\sigma_B$ [MPa] | | | | | | | | | | 497 ±44 |

## Tabelle II

| Beispiel | 21 | 22 | 23 | 24 | 25 | 26 | 27 | 28 | 29 | 30 |
|---|---|---|---|---|---|---|---|---|---|---|
| $T_g$ [°C] | 503 | 502 | 496 | 499 | 503 | 505 | 504 | 506 | 514 | 504 |
| $T_S$ [°C] | 1500 | 1500 | 1500 | 1500 | 1500 | 1500 | 1500 | 1500 | 1550 | 1550 |
| $t_S$ [min] | 60 | 60 | 60 | 60 | 60 | 60 | 60 | 60 | 60 | 60 |
| $T_1$ [°C] | 500 | 500 | 500 | 500 | 500 | 500 | 500 | 500 | 540 | 520 |
| $T_1$ [min] | 10 | 10 | 10 | 10 | 10 | 10 | 10 | 10 | 10 | 60 |
| $T_2$ [°C] | 860 | 850 | 850 | 850 | 850 | 840 | 830 | 840 | 860 | 850 |
| $T_2$ [min] | 30 | 10 | 10 | 10 | 10 | 10 | 10 | 10 | 10 | 10 |
| Hauptkristallphase | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ |
| Phasenanteil [Gew.-%] | | | | | | | | | | |
| Mitt. Länge [μm] | | | | | | | | | | |
| Mitt. Aspektverhältnis | | | | | | | | | | |
| weitere Kristallphasen | $Li_3PO_4$ | $Li_3PO_4$ | $Li_3PO_4$ | $Li_3PO_4$ | $Li_3PO_4$ | $Li_3PO_4$ | $Li_3PO_4$ | $Li_3PO_4$ $Li_2SiO_3$ | $Li_3PO_4$ | $Li_3PO_4$ |
| Abtragrate [Gew.-%·min$^{-1}$] | | | | | | | | | | |
| L* | | | 93,41 | 93,32 | 92,98 | 93,00 | 93,11 | | | 93,15 |
| a* | | | −0,68 | −0,77 | −0,72 | −0,82 | −0,78 | | | −0,81 |
| b* | | | 3,69 | 4,02 | 4,46 | 4,37 | 4,87 | | | 5,29 |
| CR | | | 45,72 | 48,26 | 43,28 | 42,50 | 43,76 | | | 47,08 |
| $K_{IC}$ [MPa m$^{0.5}$] | | 2,24 ±0,16 | | 1,90 ±0,06 | | | 1,77 ±0,12 | | 1,89 ±0,22 | |
| $\sigma_B$ [MPa] | | 223 ±82 | 600 ±55 | | 349 ±127 | 459 ±80 | | | 309 ±40 | |

EP 4 382 495 A1

Tabelle II

| Beispiel | 31 | 32 | 33 | 34 | 35 | 36 | 37 | 38 | 39 | 40 |
|---|---|---|---|---|---|---|---|---|---|---|
| $T_g$ [°C] | 506 | 508 | 514 | 513 | 511 | 520 | 513 | 508 | 512 | 508 |
| $T_S$ [°C] | 1550 | 1500 | 1550 | 1550 | 1550 | 1500 | 1550 | 1550 | 1550 | 1500 |
| $t_S$ [min] | 60 | 60 | 60 | 60 | 60 | 60 | 60 | 60 | 60 | 60 |
| $T_1$ [°C] | 560 | 500 | 540 | 520 | 560 | 500 | 540 | 520 | 560 | 500 |
| $T_1$ [min] | 10 | 10 | 10 | 60 | 10 | 10 | 10 | 60 | 10 | 10 |
| $T_2$ [°C] | 850 | 840 | 850 | 850 | 840 | 850 | 850 | 850 | 840 | 840 |
| $T_2$ [min] | 10 | 10 | 10 | 10 | 10 | 10 | 10 | 10 | 10 | 10 |
| Hauptkristallphase | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ |
| Phasenanteil [Gew.-%] | | | | | | | | | | |
| Mitt. Länge [µm] | | | | | | | | | | |
| Mitt. Aspektverhältnis | | | | | | | | | | |
| weitere Kristallphasen | $Li_3PO_4$ | $Li_3PO_4$ $Li_2SiO_3$ | $Li_3PO_4$ | $Li_3PO_4$ | $Li_3PO_4$ | $Li_3PO_4$ $Li_2SiO_3$ | $Li_3PO_4$ | $Li_3PO_4$ | $Li_3PO_4$ | $Li_3PO_4$ |
| Abtragrate [Gew.-%·min$^{-1}$] | | | | | | | | | | |
| L* | 93,17 | | | 93,27 | 93,44 | | | 93,44 | 93,45 | |
| a* | -0,85 | | | -0,93 | -0,80 | | | -0,92 | -0,81 | |
| b* | 4,47 | | | 4,66 | 3,81 | | | 4,47 | 3,72 | |
| CR | 46,09 | | | 48,50 | 48,26 | | | 50,21 | 49,63 | |
| $K_{IC}$ [MPa m$^{0.5}$] | | | 1,62 ±0,06 | | | | 1,52 ±0,09 | | | |
| $\sigma_B$ [MPa] | | | 296 ±100 | | | | 223 ±76 | | | |

EP 4 382 495 A1

Tabelle II

| Beispiel | 41 | 42 | 43 | 44 | 45 | 46 | 47 | 48 | 49 | 50 |
|---|---|---|---|---|---|---|---|---|---|---|
| $T_g$ [°C] | 507 | 502 | 499 | 503 | 500 | 500 | 498 | 492 | 497 | 500 |
| $T_s$ [°C] | 1500 | 1550 | 1500 | 1500 | 1500 | 1500 | 1500 | 1500 | 1500 | 1550 |
| $t_s$ [min] | 60 | 60 | 60 | 60 | 60 | 60 | 60 | 60 | 60 | 60 |
| $T_1$ [°C] | 500 | 500 | 500 | 520 | 520 | 520 | 540 | 540 | 540 | 530 |
| $T_1$ [min] | 10 | 10 | 10 | 10 | 10 | 10 | 10 | 10 | 10 | 30 |
| $T_2$ [°C] | 840 | 850 | 840 | 860 | 860 | 860 | 840 | 840 | 840 | 830 |
| $T_2$ [min] | 10 | 10 | 10 | 10 | 10 | 10 | 10 | 10 | 10 | 10 |
| Hauptkristallphase | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ |
| Phasenanteil [Gew.-%] | | | | | | | | | | |
| Mitt. Länge [µm] | | | | | | 0,43 | | | | |
| Mitt. Aspektverhältnis | | | | | | 2,03 | | | | |
| weitere Kristallphasen | $Li_3PO_4$ $Li_2SiO_3$ | $Li_3PO_4$ | $Li_3PO_4$ | $Li_3PO_4$ | $Li_3PO_4$ | $Li_3PO_4$ | $Li_3PO_4$ | $Li_3PO_4$ | $Li_3PO_4$ | $Li_3PO_4$ |
| Abtragrate [Gew.-%·$min^{-1}$] | | | | | | | | | | |
| L* | | | | 92,86 | 92,90 | 92,95 | 92,99 | 92,84 | 92,57 | |
| a* | | | | -1,04 | -1,04 | -1,06 | -0,82 | -0,80 | -0,69 | |
| b* | | | | 6,52 | 6,43 | 6,29 | 5,40 | 6,23 | 6,09 | |
| CR | | | | 43,80 | 44,16 | 45,98 | 40,62 | 41,53 | 39,68 | |
| $K_{IC}$ [MPa $m^{0.5}$] | | | | 1,88 ±0,12 | 1,87 ±0,04 | 1,97 ±0,31 | 1,94 ±0,11 | 1,87 ±0,09 | 2,12 ±0,22 | |
| $\sigma_B$ [MPa] | | | | 273 ±63 | 243 ±46 | 330 ±94 | 229 ±74 | 287 ±66 | 257 ±66 | |

## Tabelle II

| Beispiel | 51 | 52 | 53 | 54 | 55 | 56 | 57 | 58 | 59 | 60 |
|---|---|---|---|---|---|---|---|---|---|---|
| $T_g$ [°C] | 485 | 488 | 491 | 474 | 484 | 494 | 491 | 490 | 494 | 481 |
| $T_S$ [°C] | 1550 | 1550 | 1550 | 1550 | 1550 | 1550 | 1550 | 1550 | 1550 | 1550 |
| $t_S$ [min] | 60 | 60 | 60 | 60 | 60 | 60 | 60 | 60 | 60 | 60 |
| $T_1$ [°C] | 530 | 540 | 540 | 540 | 550 | 540 | 520 | 520 | 550 | 520 |
| $T_1$ [min] | 30 | 10 | 10 | 10 | 40 | 10 | 10 | 10 | 40 | 10 |
| $T_2$ [°C] | 830 | 840 | 830 | 820 | 820 | 850 | 840 | 830 | 830 | 840 |
| $T_2$ [min] | 10 | 10 | 10 | 10 | 10 | 10 | 10 | 10 | 10 | 10 |
| Hauptkris-tallphase | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ |
| Phasenanteil [Gew.-%] | | | | | | | | | | |
| Mitt. Länge [μm] | | | | | | | | | | |
| Mitt. Aspekt-verhältnis | | | | | | | | | | |
| weitere Kris-tallphasen | $Li_3PO_4$ | $Li_3PO_4$ | $Li_3PO_4$ | $Li_3PO_4$ | $Li_3PO_4$ | $Li_3PO_4$ | $Li_3PO_4$ | $Li_3PO_4$ | $Li_3PO_4$ | $Li_3PO_4$ |
| Abtragrate [Gew.-%·$min^{-1}$] | | | | | | | | | | |
| L* | | 94,16 | 94,02 | | | | 94,41 | 94,44 | | 94,54 |
| a* | | −0,55 | −0,62 | | | | −0,55 | −0,41 | | −0,45 |
| b* | | 3,74 | 4,07 | | | | 3,52 | 2,47 | | 2,9 |
| CR | | 72,71 | 68,16 | | | | 74,94 | 71,23 | | 78,12 |
| $K_{IC}$ [MPa m$^{0.5}$] | | | | | | | 1,78 ±0,11 | 1,87 ±0,10 | | |
| $\sigma_B$ [MPa] | | | | | | | 252 ±88 | 275 ±101 | | |

EP 4 382 495 A1

Tabelle II

| Beispiel | 61 | 62 | 63 | 64 | 65 | 66 | 67 | 68 | 69 | 70 |
|---|---|---|---|---|---|---|---|---|---|---|
| $T_g$ [°C] | 480 | 495 | 504 | 496 | 488 | 484 | 489 | 481 | 481 | 475 |
| $T_S$ [°C] | 1550 | 1550 | 1550 | 1550 | 1550 | 1550 | 1550 | 1600 | 1600 | 1500 |
| $t_S$ [min] | 60 | 60 | 60 | 60 | 60 | 60 | 60 | 60 | 60 | 60 |
| $T_1$ [°C] | 550 | 550 | 520 | 550 | 550 | 520 | 550 | 500 | 500 | 500 |
| $T_1$ [min] | 40 | 40 | 10 | 40 | 40 | 10 | 40 | 30 | 30 | 30 |
| $T_2$ [°C] | 870 | 830 | 850 | 830 | 830 | 830 | 830 | 850 | 840 | 830 |
| $T_2$ [min] | 10 | 10 | 10 | 10 | 10 | 10 | 10 | 5 | 1 | 30 |
| Hauptkristallphase | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ |
| Phasenanteil [Gew.-%] | 24,2 | 27,9 | | | | | | 35,3 | 33,4 | 22,9 |
| Mitt. Länge [µm] | 0,51 | 0,20 | | | | | | 0,19 | | 0,34 |
| Mitt. Aspektverhältnis | 2,28 | 1,87 | | | | | | 2,26 | | 2,53 |
| weitere Kristallphasen | $Li_3PO_4$ | $Li_3PO_4$ | $Li_3PO_4$ | $Li_3PO_4$ | $Li_3PO_4$ | $Li_3PO_4$ | $Li_3PO_4$ | $Li_3PO_4$ | $Li_3PO_4$ | $Li_3PO_4$ Cristobalit Quarz |
| Abtragrate [Gew.-%·min$^{-1}$] | 13,89 | 33,99 | | | | | | | | |
| L* | | | 93,73 | | 94,02 | 94,18 | | | | |
| a* | | | -0,75 | | -0,51 | -0,53 | | | | |
| b* | | | 5,22 | | 4,05 | 4,08 | | | | |
| CR | | | 60,72 | | 69,66 | 73,7 | | | | |
| $K_{IC}$ [MPa m$^{0.5}$] | | | | | | | | | | |
| $\sigma_B$ [MPa] | | | | | | | | | | |

Tabelle II

| Beispiel | 71 | 72 | 73 | 74 | 75 | 76 | 77 | VGB |
|---|---|---|---|---|---|---|---|---|
| $T_g$ [°C] | 504 | 513 | 508 | 496 | 508 | 508 | 501 | |
| $T_s$ [°C] | 1550 | 1550 | 1550 | 1550 | 1550 | 1550 | 1550 | |
| $t_s$ [min] | 60 | 60 | 60 | 60 | 60 | 60 | 60 | |
| $T_1$ [°C] | 600 | 600 | 600 | 600 | 600 | 530 | 530 | |
| $T_1$ [min] | 30 | 30 | 30 | 30 | 30 | 10 | 10 | |
| $T_2$ [°C] | 850 | 850 | 850 | 850 | 850 | 850 | 850 | |
| $T_2$ [min] | 30 | 30 | 30 | 30 | 30 | 30 | 30 | |
| Hauptkristallphase | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ |
| Phasenanteil [Gew.-%] | | | | | | | | |
| Mitt. Länge [μm] | | | | | | | | |
| Mitt. Aspektverhältnis | | | | | | | | |
| weitere Kristallphasen | $Li_3PO_4$ | $Li_3PO_4$ | $Li_3PO_4$ | $Li_3PO_4$ | $Li_3PO_4$ | $Li_3PO_4$ $Li_2SiO_3$ | $Li_3PO_4$ $Li_2SiO_3$ | $Li_3PO_4$ |
| Abtragrate [Gew.-%·min⁻¹] | | | | | | | | 7,4 |
| L* | | | | | | | | |
| a* | | | | | | | | |
| b* | | | | | | | | |
| CR | | | | | | | | |
| $K_{Ic}$ [MPa m$^{0.5}$] | | | | | | | | |
| $\sigma_B$ [MPa] | | | | | | | | |

**Patentansprüche**

1. Lithiumsilikat-Glaskeramik, die Lithiumdisilikat als Hauptkristallphase aufweist und nicht mehr als 40 Gew.-% an Lithiumdisilikat-Kristallen enthält.

2. Glaskeramik nach Anspruch 1, die nicht mehr als 35 Gew.-%, bevorzugt nicht mehr als 32 Gew.-%, weiter bevorzugt nicht mehr als 30 Gew.-% und besonders bevorzugt nicht mehr als 28 Gew.-%, und insbesondere 10 bis 40 Gew.-%, bevorzugt 15 bis 35 Gew.-%, weiter bevorzugt 20 bis 32 Gew.-% und besonders bevorzugt 22 bis 30 Gew.-% an Lithiumdisilikat-Kristallen enthält.

3. Glaskeramik nach Anspruch 1 oder 2, bei der die Lithiumdisilikatkristalle eine mittlere Länge im Bereich von 10 bis 1000 nm, bevorzugt im Bereich von 50 bis 750 nm, besonders bevorzugt im Bereich von 100 bis 500 nm und ganz besonders bevorzugt im Bereich von 150 bis 250 nm, und ein Aspektverhältnis im Bereich von 1,0 bis 5,0, bevorzugt im Bereich von 1,25 bis 3,0, besonders bevorzugt im Bereich von 1,5 bis 2,5 und ganz besonders bevorzugt im Bereich von 1,75 bis 2,0 aufweisen.

4. Glaskeramik nach einem der Ansprüche 1 bis 3, die 62,0 bis 80,0, bevorzugt 64,0 bis 75,0 und besonders bevorzugt 65,0 bis 73,0 Gew.-% $SiO_2$ enthält.

5. Glaskeramik nach einem der Ansprüche 1 bis 4, die 7,0 bis 13,0, bevorzugt 9,0 bis 12,5 und besonders bevorzugt 10,0 bis 12,0 Gew.-% $Li_2O$ enthält.

6. Glaskeramik nach einem der Ansprüche 1 bis 5, die 2,0 bis 12,0, bevorzugt 3,0 bis 10,0 und besonders bevorzugt 5,0 bis 9,0 Gew.-% weiteres Oxid einwertiger Elemente $Me^I_2O$ enthält, wobei $Me^I_2O$ aus $Na_2O$, $K_2O$, $Rb_2O$, $Cs_2O$ und Mischungen davon ausgewählt ist und vorzugsweise $K_2O$ ist.

7. Glaskeramik nach einem der Ansprüche 1 bis 6, die 3,0 bis 12,0, bevorzugt 3,0 bis 10,0, besonders bevorzugt 3,0 bis 9,0 und ganz besonders bevorzugt 4,0 bis 7,0 Gew.-% $Al_2O_3$ enthält.

8. Glaskeramik nach einem der Ansprüche 1 bis 7, die 0,5 bis 10,0, bevorzugt 1,0 bis 8,0, weiter bevorzugt 1,5 bis 6,0, besonders bevorzugt 1,8 bis 5,0 und ganz besonders bevorzugt 2,0 bis 3,0 Gew.-% $P_2O_5$ enthält.

9. Glaskeramik nach einem der Ansprüche 1 bis 8, die mindestens eine und bevorzugt alle der folgenden Komponenten in den angegebenen Mengen enthält:

| Komponente | Gew.-% |
|---|---|
| $SiO_2$ | 62,0 bis 75,0 |
| $Li_2O$ | 7, 0 bis 12,0 |
| $Me^I_2O$ | 2,0 bis 12,0, insbesondere 5,0 bis 10,0 |
| $Al_2O_3$ | 3,0 bis 12,0 |
| $P_2O_5$ | 0, 5 bis 5, 0 |
| $Me^{II}O$ | 0 bis 8, 0 |
| $Me^{III}_2O_3$ | 0 bis 8, 0 |
| $Me^{IV}O_2$ | 0 bis 12,0 |
| $Me^V_2O_5$ | 0 bis 9, 0 |
| $Me^{VI}O_3$ | 0 bis 3,0 |
| Fluor | 0 bis 2,0, |

wobei

$Me^I_2O$ ausgewählt ist aus $Na_2O$, $K_2O$, $Rb_2O$, $Cs_2O$ und Mischungen davon,
$Me^{II}O$ ausgewählt ist aus $MgO$, $CaO$, $SrO$, $ZnO$ und Mischungen davon,
$Me^{III}_2O_3$ ausgewählt ist aus $B_2O_3$, $Y_2O_3$, $La_2O_3$, $Ga_2O_3$, $In_2O_3$ und Mischungen davon,
$Me^{IV}O_2$ ausgewählt ist aus $TiO_2$, $ZrO_2$, $GeO_2$, $SnO_2$, $CeO_2$ und Mischungen davon,
$Me^V_2O_5$ ausgewählt ist aus $V_2O_5$, $Nb_2O_5$, $Ta_2O_5$ und Mischungen davon und
$Me^{VI}O_3$ ausgewählt ist aus $MoO_3$, $WO_3$ und Mischungen davon.

10. Glaskeramik nach einem der Ansprüche 1 bis 9, bei der das molare Verhältnis von $SiO_2$ zu $Li_2O$ im Bereich von 2,5 bis 4,0, bevorzugt im Bereich von 2,8 bis 3,8 und besonders bevorzugt im Bereich von 3,0 bis 3,6 liegt.

11. Glaskeramik nach einem der Ansprüche 1 bis 10, die weniger als 12 Gew.-%, insbesondere weniger als 10 Gew.-%, bevorzugt weniger als 8 Gew.-% und besonders bevorzugt weniger als 5 Gew.-% an Nebenkristallphasen, insbesondere Lithiummetasilikat-Kristallen, Lithiumphosphat-Kristallen, $SiO_2$-Kristallen wie Quarz-Kristallen oder Cristobalit-Kristallen, $SiO_2$-Mischkristallen wie Quarz-Mischkristallen, Cristobalit-Mischkristallen oder Lithiumalumi-

niumsilikat-Kristallen, oder $ZrO_2$-Kristallen enthält.

12. Glaskeramik nach einem der Ansprüche 1 bis 11, die weniger als 10 Gew.-%, insbesondere weniger als 7 Gew.-%, bevorzugt weniger als 5 Gew.-%, besonders bevorzugt weniger als 3 Gew.-% und ganz besonders bevorzugt weniger als 1 Gew.-% an Quarz-Kristallen und/oder Quarz-Mischkristallen enthält.

13. Ausgangsglas, das die Komponenten der Glaskeramik nach einem der Ansprüche 1 bis 12 enthält und insbesondere Keime für die Bildung von Lithiumdisilikatkristallen enthält.

14. Glaskeramik nach einem der Ansprüche 1 bis 12 oder Ausgangsglas nach Anspruch 13, wobei die Glaskeramik und das Ausgangsglas in Form von einem Pulver, einem Granulat, einem Rohling oder einer dentalen Restauration vorliegen.

15. Verfahren zur Herstellung der Glaskeramik gemäß einem der Ansprüche 1 bis 12 oder 14, bei dem das Ausgangsglas gemäß Anspruch 13 oder 14 mindestens einer Wärmebehandlung bei einer Temperatur von 400 bis 1000°C für eine Dauer von insbesondere 1 bis 120 min, bevorzugt 5 bis 90 min und besonders bevorzugt 10 bis 60 min, unterzogen wird.

16. Verfahren nach Anspruch 15, bei dem

(a) das Ausgangsglas einer Wärmebehandlung bei einer Temperatur von 400 bis 650°C, bevorzugt 450 bis 600°C und besonders bevorzugt 480 bis 580°C, für eine Dauer von insbesondere 1 bis 240 min, bevorzugt 5 bis 120 min und besonders bevorzugt 10 bis 60 min, unterworfen wird, um Ausgangsglas mit Keimen zu bilden, und

(b) das Ausgangsglas mit Keimen einer Wärmebehandlung bei einer Temperatur von 700 bis 1000°C, bevorzugt 750 bis 950°C und besonders bevorzugt 800 bis 900°C, für eine Dauer von insbesondere 1 bis 120 min, bevorzugt 2 bis 90 min, besonders bevorzugt 5 bis 60 min und ganz besonders bevorzugt 10 bis 30 min, unterworfen wird, um die Glaskeramik zu bilden.

17. Verwendung der Glaskeramik gemäß einem der Ansprüche 1 bis 12 oder 14 oder des Ausgangsglases gemäß Anspruch 13 oder 14 als Dentalmaterial, bevorzugt zur Beschichtung einer dentalen Restauration und besonders bevorzugt zur Herstellung einer dentalen Restauration.

18. Verwendung zur Herstellung einer dentalen Restauration nach Anspruch 17, wobei der Glaskeramik durch Verpressen oder maschinelle Bearbeitung die Form der gewünschten dentalen Restauration, insbesondere Brücke, Inlay, Onlay, Veneer, Abutment, Teilkrone, Krone oder Schale, gegeben wird.

19. Verwendung nach Anspruch 17 oder 18, bei der die Glaskeramik einer Wärmebehandlung bei einer Temperatur von 700 bis 1000°C, bevorzugt 750 bis 950°C und besonders bevorzugt 800 bis 900°C, insbesondere für eine Dauer von 1 bis 90 Minuten, bevorzugt 2 bis 60 Minuten, besonders bevorzugt 5 bis 30 Minuten und ganz besonders bevorzugt 10 bis 15 Minuten, unterworfen wird.

20. Verfahren zur Herstellung einer dentalen Restauration, insbesondere Brücke, Inlay, Onlay, Veneer, Abutment, Teilkrone, Krone oder Schale, bei dem der Glaskeramik gemäß einem der Ansprüche 1 bis 12 oder 14 oder dem Ausgangsglas gemäß Anspruch 13 oder 14 durch Verpressen oder maschinelle Bearbeitung, insbesondere im Rahmen eines CAD/CAM-Verfahrens, die Form der gewünschten dentalen Restauration gegeben wird.

Europäisches Patentamt
European Patent Office
Office européen des brevets

# EUROPÄISCHER RECHERCHENBERICHT

**Nummer der Anmeldung**

EP 22 21 2273

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 3 135 641 A1 (IVOCLAR VIVADENT AG [LI]) 1. März 2017 (2017-03-01) * Absätze [0001], [0041], [0056] – [0062], [0064] – [0071]; Beispiele 5,7-10,12-20,25,33; Tabelle 1 * ----- | 1–9, 13–20 | INV. C03C3/097 C03C4/00 C03C10/04 C03C10/00 |
| X | WO 2020/025795 A1 (IVOCLAR VIVADENT AG [LI]) 6. Februar 2020 (2020-02-06) * Seite 19, Zeile 1 – Zeile 22 * * Seite 26, Zeile 15 – Seite 28, Zeile 12 * ----- | 1–3,10, 13–15, 17–20 | |
| X | US 3 816 704 A (BOROM M ET AL) 11. Juni 1974 (1974-06-11) * Abbildung 4b; Beispiel 1 * ----- | 1,2,4, 6–8, 10–14 | |
| X | US 2021/214269 A1 (YUKI KEN [JP]) 15. Juli 2021 (2021-07-15) * Beispiel 1 * ----- | 1,3–14 | |
| X | JP 2001 126236 A (NGK INSULATORS LTD) 11. Mai 2001 (2001-05-11) * das ganze Dokument * ----- | 1,3–8, 10,13,14 | **RECHERCHIERTE SACHGEBIETE (IPC)** C03C |

Der vorliegende Recherchenbericht wurde für alle Patentansprüche erstellt

| Recherchenort | Abschlußdatum der Recherche | Prüfer |
|---|---|---|
| München | 8. Mai 2023 | King, Ruth |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................................
& : Mitglied der gleichen Patentfamilie, übereinstimmendes Dokument

EPO FORM 1503 03.82 (P04C03)

**EP 4 382 495 A1**

**ANHANG ZUM EUROPÄISCHEN RECHERCHENBERICHT**
**ÜBER DIE EUROPÄISCHE PATENTANMELDUNG NR.**   EP 22 21 2273

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-05-2023

| Im Recherchenbericht angeführtes Patentdokument | Datum der Veröffentlichung | Mitglied(er) der Patentfamilie | Datum der Veröffentlichung |
|---|---|---|---|
| EP 3135641 A1 | 01-03-2017 | CA 2995309 A1 | 02-03-2017 |
| | | CN 108025949 A | 11-05-2018 |
| | | DE 202015009943 U1 | 12-10-2021 |
| | | EP 3135641 A1 | 01-03-2017 |
| | | JP 6907192 B2 | 21-07-2021 |
| | | JP 2018526317 A | 13-09-2018 |
| | | JP 2021066658 A | 30-04-2021 |
| | | KR 20180043335 A | 27-04-2018 |
| | | KR 20200086382 A | 16-07-2020 |
| | | US 2018244564 A1 | 30-08-2018 |
| | | US 2020079685 A1 | 12-03-2020 |
| | | US 2022227663 A1 | 21-07-2022 |
| | | WO 2017032745 A1 | 02-03-2017 |
| WO 2020025795 A1 | 06-02-2020 | CN 112272544 A | 26-01-2021 |
| | | EP 3829482 A1 | 09-06-2021 |
| | | JP 2021531839 A | 25-11-2021 |
| | | KR 20210039333 A | 09-04-2021 |
| | | US 2021196437 A1 | 01-07-2021 |
| | | WO 2020025795 A1 | 06-02-2020 |
| US 3816704 A | 11-06-1974 | AU 5233773 A | 22-08-1974 |
| | | DE 2313347 A1 | 04-10-1973 |
| | | FR 2176901 A1 | 02-11-1973 |
| | | US 3816704 A | 11-06-1974 |
| US 2021214269 A1 | 15-07-2021 | CN 112166091 A | 01-01-2021 |
| | | CN 115196874 A | 18-10-2022 |
| | | JP WO2019230889 A1 | 10-06-2021 |
| | | US 2021214269 A1 | 15-07-2021 |
| | | WO 2019230889 A1 | 05-12-2019 |
| JP 2001126236 A | 11-05-2001 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9532678 A2
- EP 0827941 A1
- EP 0916625 A1
- EP 1505041 A1
- EP 1688398 A1
- EP 231773 A

**In der Beschreibung aufgeführte Nicht-Patentliteratur**

- Gläser und Glaskeramiken im System MgO-Al2O3-SiO2 mit ZrO2 als Keimbildner. **VON M. DITTMER.** Dissertation. Universität Jena, 2011